(19) Europäisches Patentamt
European Patent Office
Office européen des brevets

(11) Veröffentlichungsnummer: **0 080 602**
**A1**

# (12) EUROPÄISCHE PATENTANMELDUNG

(21) Anmeldenummer: **82110054.2**

(22) Anmeldetag: **30.10.82**

(51) Int. Cl.³: **C 07 D 401/12**
A 61 K 31/44, A 61 K 31/415

(30) Priorität: **05.11.81 CH 7080/81**
**05.11.81 CH 7081/81**

(43) Veröffentlichungstag der Anmeldung:
**08.06.83 Patentblatt 83/23**

(84) Benannte Vertragsstaaten:
**AT BE CH DE FR GB IT LI LU NL SE**

(71) Anmelder: **Byk Gulden Lomberg Chemische Fabrik GmbH**
**Byk-Gulden-Strasse 2**
**D-7750 Konstanz(DE)**

(72) Erfinder: **Senn-Bilfinger, Jörg, Dr.**
**Renkenweg 12**
**D-7750 Konstanz(DE)**

(72) Erfinder: **Schaefer, Hartmann, Dr.**
**Zum Purren 27**
**D-7750 Konstanz 16(DE)**

(72) Erfinder: **Figala, Volker, Dr.**
**Am Hochfirst 2**
**D-7753 Allensbach 4(DE)**

(72) Erfinder: **Klemm, Kurt, Prof, Dr.**
**Im Weinberg 2**
**D-7753 Allensbach(DE)**

(72) Erfinder: **Rainer, Georg, Dr.**
**Josef-Anton-Feuchtmayerstrasse 7**
**D-7750 Konstanz(DE)**

(72) Erfinder: **Riedel, Richard, Dr.**
**Salmannsweilergasse 36**
**D-7750 Konstanz(DE)**

(72) Erfinder: **Schudt, Christian, Dr.**
**Hoheneggstrasse 102**
**D-7750 Konstanz(DE)**

(72) Erfinder: **Simon, Wolfgang, Dr.**
**Seestrasse 31a**
**D-7750 Konstanz(DE)**

(54) **Substituierte Benzimidazole, Verfahren zu ihrer Herstellung, ihre Anwendung und sie enthaltende Arzneimittel.**

(57) Substituierte Benzimidazole der allgemeinen Formel I

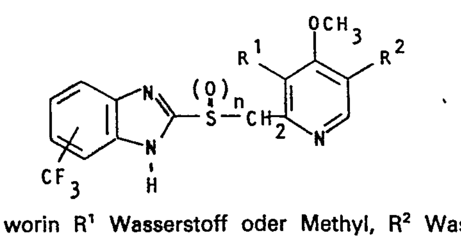

(I),

worin $R^1$ Wasserstoff oder Methyl, $R^2$ Wasserstoff oder Methyl und n 0 oder 1 bedeutet und ihre Salze sind neue Verbindungen, die die Magensäuresekretion hemmen und die eine ausgezeichnete Magen- und Darmschutzwirkung bei Warmblütern zeigen.

EP 0 080 602 A1

Croydon Printing Company Ltd

# Substituierte Benzimidazole, Verfahren zu ihrer Herstellung, ihre Anwendung und sie enthaltende Arzneimittel

## Anwendungsgebiet der Erfindung

Die Erfindung betrifft neue substituierte Benzimidazole, Verfahren zu ihrer Herstellung, ihre Anwendung und sie enthaltende Arzneimittel.

Die erfindungsgemäßen Verbindungen werden in der pharmazeutischen Industrie als Zwischenprodukte und zur Herstellung von Medikamenten verwendet.

## Bekannter technischer Hintergrund

In der Deutschen Offenlegungsschrift DE-OS 18 04 450 werden Benzazol-Derivate beschrieben, die tuberkulostatische, insektizide, fungizide, antivirale, anthelmintische und entzündungshemmende Wirkung aufweisen sollen. - In der DE-OS 25 04 252 werden 2-Benzimidazolyl-2-pyridyl-sulfide beansprucht, die teils eine magensäuresekretionshemmende, teils eine magensäuresekretionsstimulierende Wirkung besitzen sollen. - In der DE-OS 25 48 340 werden 2-Benzimidazolyl-2-pyridyl-sulfoxide beschrieben, die die exogen oder endogen stimulierte Magensäure-sekretion hemmen sollen. - In der Europäischen Patentschrift EP-B1 0 005 129 werden ebenfalls 2-Benzimidazolyl-2 pyridyl-sulfoxide beschrieben, die in pharmazeutischen Präparaten zur Hemmung der Magensäuresekretion Verwendung finden sollen. - Die für die Herstellung der obenerwähnten Sulfoxide einsetzbaren entsprechend substituierten Sulfide werden in der DE-OS 25 48 340 und in der EP-B1 0 005 129 ausschließlich als Zwischenprodukt ohne Angabe einer pharmakologischen Wirksamkeit beschrieben. -

In der veröffentlichten Europäischen Patentanmeldung EP-A1 0 045 200 werden substituierte Heterocyclylalkylsulfinyl-benzimidazole beschrieben, die bei der Bekämpfung oder Prävention bestimmter gastrointestinaler Entzündungskrankheiten verwendet werden sollen.

Beschreibung der Erfindung

Überraschenderweise wurde nun gefunden, daß die unten näher beschriebenen erfindungsgemäßen 2-Benzimidazolyl-2-pyridylmethyl-sulfide und -sulfoxide interessante und unerwartete Eigenschaften aufweisen, durch die sie sich von bekannten Verbindungen in vorteilhafter Weise unterscheiden.

Gegenstand der Erfindung sind neue substituierte Benzimidazole der allgemeinen Formel I

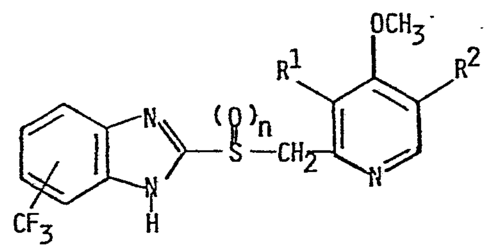

(I),

worin

$R^1$ Wasserstoff oder Methyl und

$R^2$ Wasserstoff oder Methyl bedeutet und

n für die Zahlen 0 oder 1 steht,

sowie die Salze dieser Verbindungen.

Als Salze kommen für Verbindungen der Formel I, in denen n die Zahl 0 bedeutet (Sulfide), alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat [2-(4-Hydroxybenzoyl)-benzoat], Fendizoat (o-[(2'-Hydroxy-4-biphenylyl)-carbonyl]-benzoat), Butyrat,

Sulfosalicylat, Maleat, Laurat, Malat, Fumarat, Succinat, Oxalat, Tartrat, Amsonat (4,4'-Diaminostilben-2,2'-disulfonat), Embonat [4,4'-Methylen-bis-(3-hydroxy-2-naphthoat)], Metembonat [4,4'-Methylen-bis-(3-methoxy-2-naphthoat)], Stearat, Tosilat (p-Toluolsulfonat), 2-Hydroxy-3-naphthoat, 3-Hydroxy-2-naphthoat, Mesilat (Methansulfonat).

Von Verbindungen der Formel I, in denen n die Zahl 1 bedeutet (Sulfoxide), lassen sich ebenfalls die oben erwähnten Säureadditionssalze herstellen, jedoch besitzen diese in wäßriger Lösung nicht die gleiche Stabilität wie die Salze der Sulfide. Von den Sulfoxiden lassen sich andererseits durch Umsetzung mit geeigneten Deprotonierungsmitteln (z.B. anorganischen oder organischen Basen) basische Salze herstellen, die ebenfalls Gegenstand der Erfindung sind.

Eine Ausgestaltung der Erfindung sind substituierte Benzimidazole der allgemeinen Formel $I^a$

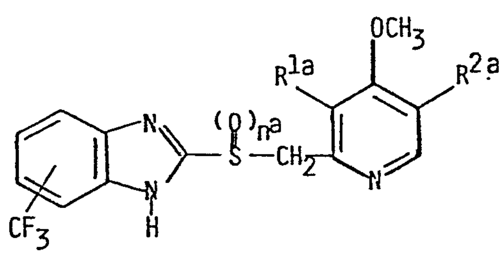

$(I^a)$,

worin
$R^{1a}$ die Bedeutung von $R^1$ und
$R^{2a}$ die Bedeutung von $R^2$ hat und
$n^a$ die Zahl 0 bedeutet,
und die Salze dieser Verbindungen.

Eine weitere Ausgestaltung der Erfindung sind substituierte Benzimidazole der allgemeinen Formel $I^b$

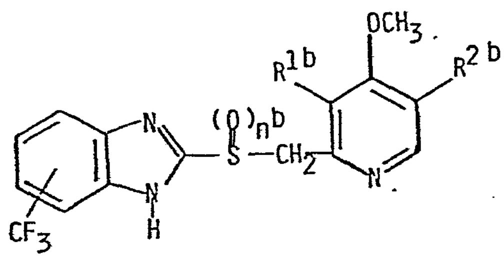

$(I^b)$,

worin

$R^{1b}$ die Bedeutung von $R^1$ und

$R^{2b}$ die Bedeutung von $R^2$ hat und

$n^b$ die Zahl 1 bedeutet,

und die Salze dieser Verbindungen.

Als erfindungsgemäße Verbindungen seien genannt:

4-Trifluormethyl-2-[(4-methoxy-2-pyridylmethyl)thio]-(1H)-benzimidazol

4-Trifluormethyl-2-[(4-methoxy-3-methyl-2-pyridylmethyl)thio]-(1H)-benzimidazol

4-Trifluormethyl-2-[(4-methoxy-5-methyl-2-pyridylmethyl)thio]-(1H)-benzimidazol

4-Trifluormethyl-2-[(4-methoxy-3,5-dimethyl-2-pyridylmethyl)thio]-(1H)-benzimidazol

5-Trifluormethyl-2-[(4-methoxy-2-pyridylmethyl)thio]-(1H)-benzimidazol

5-Trifluormethyl-2-[(4-methoxy-3-methyl-2-pyridylmethyl)thio]-(1H)-benzimidazol

5-Trifluormethyl-2-[(4-methoxy-5-methyl-2-pyridylmethyl)thio]-(1H)-benzimidazol

5-Trifluormethyl-2-[(2-methoxy-3,5-dimethyl-2-pyridylmethyl)thio]-(1H)-benzimidazol

4-Trifluormethyl-2-[(4-methoxy-2-pyridylmethyl)sulfinyl]-(1H)-benzimidazol

4-Trifluormethyl-2-[(4-methoxy-3-methyl-2-pyridylmethyl)sulfinyl]-(1H)-benzimidazol

4-Trifluormethyl-2-[(4-methoxy-5-methyl-2-pyridylmethyl)sulfinyl]-(1H) benzimidazol

4-Trifluormethyl-2-[(4-methoxy-3,5-dimethyl-2-pyridylmethyl)sulfinyl]-(1H)-benzimidazol
5-Trifluormethyl-2-[(4-methoxy-2-pyridylmethyl)sulfinyl]-(1H)-benzimidazol
5-Trifluormethyl-2-[(4-methoxy-3-methyl-2-pyridylmethyl)sulfinyl]-(1H)-benzimidazol
5-Trifluormethyl-2-[(4-methoxy-5-methyl-2-pyridylmethyl)sulfinyl]-(1H)-benzimidazol
5-Trifluormethyl-2-[(4-methoxy-3,5-dimethyl-2-pyridylmethyl)sulfinyl]-(1H)-benzimidazol
und ihre Salze.

Als besonders bevorzugte Verbindungen sind hervorzuheben: 5-Trifluormethyl-2-[(4-methoxy-3-methyl-2-pyridylmethyl)thio]-(1H)-benzimidazol, 5-Trifluormethyl-2-[(4-methoxy-2-pyridylmethyl)sulfinyl]-(1H)-benzimidazol und 5-Trifluormethyl-2-[(4-methoxy-3,5-dimethyl-2-pyridylmethyl)sulfinyl]-(1H)-benzimidazol und ihre pharmakologisch verträglichen Salze.

Bedingt durch die Tautomerie im Imidazolring ist die 4- bzw. 5-Substitution im Benzimidazol mit der 7- bzw. 6-Substitution identisch.

Ein weiterer Gegenstand der Erfindung ist ein Verfahren zur Herstellung der substituierten Benzimidazole der Formel I, worin $R^1$, $R^2$ und n die oben angegebene Bedeutung haben, und ihrer Salze.

Das Verfahren ist dadurch gekennzeichnet, daß man
a) Mercaptobenzimidazole der Formel II mit Picolinderivaten III,

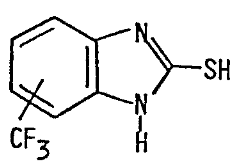 (II), 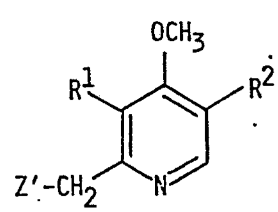 (III),

oder

b) Benzimidazole der Formel IV mit Mercaptopicolinen V,

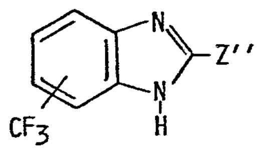 (IV), 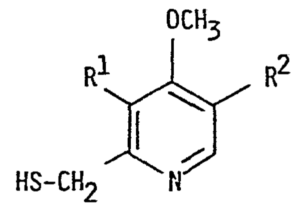 (V),

oder

c) o-Phenylendiamine der Formel VI mit Ameisensäurederivaten VII

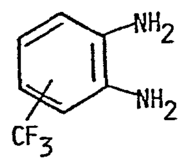 (VI), 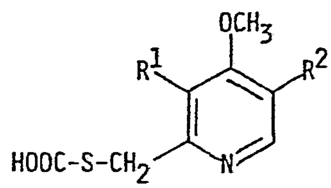 (VII),

umsetzt und gegebenenfalls anschließend die nach a), b) oder c) erhaltenen 2-Benzimidazolyl-2-pyridyl-sulfide der Formel VIII

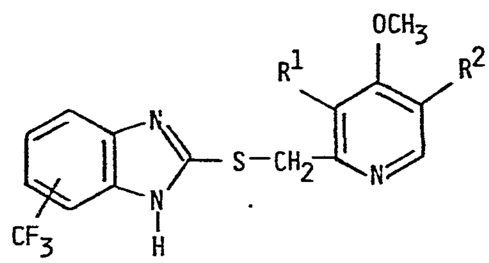 (VIII),

oxidiert und/oder in die Salze überführt,

oder daß man

d) Benzimidazole der Formel IX mit Pyridinderivaten X

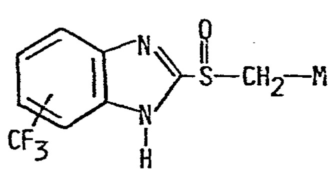 (IX), 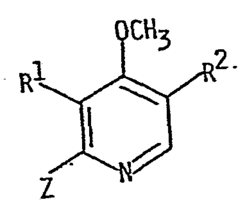 (X),

oder

e) Sulfinylderivate der Formel XI mit 2-Picolinderivaten XII

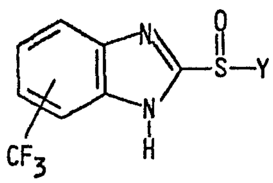 (XI) 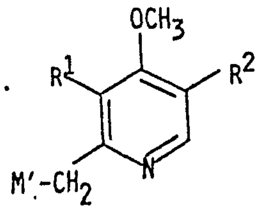 (XII)

umsetzt und gegebenenfalls anschließend in die Salze überführt, wobei Y, Z, Z' und Z'' geeignete Abgangsgruppen darstellen, M für ein Alkalimetallatom (Li, Na oder K) steht, M' für das Äquivalent eines Metallatoms steht und $R^1$, $R^2$ und n die oben angegebenen Bedeutungen haben.

Die Herstellungsverfahren a), b) und c) führen zu den erfindungsgemäßen Sulfiden, die Oxidation der Verbindungen VIII und die Verfahren d) und e) liefern die erfindungsgemäßen Sulfoxide.

Welche Abgangsgruppen Y, Z, Z' bzw. Z'' geeignet sind, ist dem Fachmann aufgrund seines Fachwissens geläufig. Eine geeignete Abgangsgruppe Y ist beispielsweise eine Gruppe, die zusammen mit der Sulfinylgruppe, an die sie gebunden ist, ein reaktives Sulfinsäurederivat bildet. Als geeignete Abgangsgruppe Y seien beispielsweise Alkoxy-, Dialkylamino- oder Alkylmercaptogruppen genannt. Als geeignete Abgangsgruppen Z, Z' bzw. Z'' seien beispielsweise Halogenatome, insbesondere Chloratome, oder durch Veresterung (z.B. mit p-Toluolsulfonsäure) aktivierte Hydroxylgruppen genannt. Das Äquivalent eines Metallatoms M' ist beispielsweise ein Alkalimetallatom (Li, Na oder K), oder ein Erdalkalimetallatom (z.B. Mg) das durch ein Halogenatom (z.B. Br, Grignard-Reagenz) substituiert ist, oder irgendein anderes, gegebenenfalls substituiertes Metallatom, von dem bekannt ist, daß es bei Substitutionsreaktionen metallorganischer Verbindungen wie die obenerwähnten Metalle reagiert.

Die Umsetzung von II mit III erfolgt in an sich bekannter Weise in geeigneten, vorzugsweise polaren Lösungsmitteln (wie Methanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide,

wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze entstehen. Die Reaktionstemperatur kann zwischen $0^o$ und $150^oC$ liegen, wobei Temperaturen zwischen $50^oC$ und $100^oC$ - insbesondere die Siedetemperatur der verwendeten Lösungsmittel - bevorzugt sind.

Bei der Umsetzung von IV mit V, die in an sich bekannter Weise erfolgt, kommen ähnliche Reaktionsbedingungen wie bei der Umsetzung von II mit III zur Anwendung.

Die Reaktion von VI mit VII wird bevorzugt in polaren, gegebenenfalls wasserhaltigen Lösungsmitteln in Gegenwart einer starken Säure, z.B. Salzsäure, insbesondere bei der Siedetemperatur des verwendeten Lösungsmittels durchgeführt.

Die Oxidation der Sulfide VIII erfolgt in an sich bekannter Weise und unter den Bedingungen, wie sie dem Fachmann für die Oxidation von Sulfiden zu Sulfoxiden geläufig sind [siehe hierzu z.B. J. Drabowicz und M. Mikolajczyk, Organic preparations and procedures int. 14(1-2), 45-89 (1982)]. Als Oxidationsmittel kommen alle für die Oxidation von Sulfiden üblicherweise verwendeten Reagenzien in Frage, insbesondere Peroxysäuren, wie z.B. Peroxyessigsäure, Trifluorperoxyessigsäure, 3,5-Dinitroperoxybenzoesäure, Peroxymaleinsäure oder bevorzugt m-Chlorperoxybenzoesäure. Die Reaktion wird zweckmäßigerweise in inerten Lösungsmitteln, z.B. aromatischen oder chlorierten Kohlenwasserstoffen, wie Benzol, Toluol, Dichlormethan oder Chloroform durchgeführt. Die Reaktionstemperatur liegt (je nach Reaktivität des Oxidationsmittels und Verdünnungsgrad) zwischen $-70^oC$ und der Siedetemperatur des verwendeten Lösungsmittels, bevorzugt jedoch zwischen $-30^oC$ und $+20^oC$. Als sehr vorteilhaft hat sich auch die Oxidation mit Halogenen bzw. mit Hypohalogeniten (z.B. mit wäßriger Natriumhypochloritlösung) erwiesen, die zweckmäßigerweise bei Temperaturen zwischen $0^o$ und $30^oC$ durchgeführt wird.

Die Umsetzung von IX mit X erfolgt bevorzugt in inerten Lösungsmitteln, wie sie auch für die Reaktion von Enolationen mit Alkylierungsmitteln üblicherweise verwendet werden. Beispielsweise seien aromatische Lösungsmittel wie Benzol oder Toluol genannt. Die Reaktionstemperatur liegt (je nach Art des Alkalimetallatoms M und der Abgangsgruppe Z) in der Regel zwischen $0^{o}$ und $120^{o}C$, wobei die Siedetemperatur des verwendeten Lösungsmittels bevorzugt ist. Beispielsweise [wenn M Li (Lithium) und Z Cl (Chlor) darstellt und die Umsetzung in Benzol durchgeführt wird] ist die Siedetemperatur von Benzol ($80^{o}C$) bevorzugt.

Die Verbindungen XI werden mit den Verbindungen XII in an sich bekannter Weise umgesetzt, wie sie dem Fachmann für die Reaktion metallorganischer Verbindungen geläufig ist.

Je nach Art der Ausgangsverbindungen, die gegebenenfalls auch in Form ihrer Salze eingesetzt werden können, und in Abhängigkeit von den Reaktionsbedingungen werden die erfindungsgemäßen Verbindungen zunächst entweder als solche oder in Form ihrer Salze gewonnen.

Im übrigen erhält man die Salze durch Auflösen der freien Verbindungen in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base - gegebenenfalls in der genau berechneten stöchiometrischen Menge - anschließend zugegeben wird.

Die Salze werden durch Filtrieren, Umfällen, Ausfällen oder durch Verdampfen des Lösungsmittels gewonnen.

Erhaltene Salze können durch Alkalisieren bzw. Ansäuern, z. B. mit wäßrigem Natriumhydrogencarbonat bzw. mit verdünnter Salzsäure, in die freien Verbindungen umgewandelt werden, welche wiederum in die Salze übergeführt werden können. Auf diese Weise lassen sich die Verbindungen reinigen, oder es lassen sich pharmakologisch nicht verträgliche Salze in pharmakologisch verträgliche Salze umwandeln.

Die erfindungsgemäßen Sulfoxide sind optisch aktive Verbindungen. Die Erfindung umfaßt daher sowohl die Enantiomeren als auch ihre Mischungen und Racemate. Die Enantiomeren können in an sich bekannter Weise (beispielsweise durch Herstellung und Trennung entsprechender diastereoisomerer Verbindungen) separiert werden. Die Enantiomeren können aber auch durch asymmetrische Synthese, beispielsweise durch Reaktion optisch aktiver reiner Verbindungen XI oder diastereoisomer reiner Verbindungen XI mit Verbindungen XII hergestellt werden [siehe hierzu K.K.Andersen, Tetrahedron Lett., 93 (1962)].
Die erfindungsgemäßen Verbindungen werden bevorzugt durch Umsetzung von II mit III und gegebenenfalls anschließende Oxidation des entstandenen Sulfids VIII synthetisiert.

Die Verbindungen der Formeln II - VII sowie IX - XII sind entweder bekannt, oder sie können nach bekannten Vorschriften analog hergestellt werden. So erhält man beispielsweise die Verbindungen II durch Umsetzung von Verbindungen VI [E. Pouterman u.A. Girardet, Helvet. Chim. Acta 30, 107 (1947)] mit Kohlendisulfid. Die Verbindungen III können gemäß O.E.Schulz u. S. Fedders, Arch. Pharm. (Weinheim) 310, 128-136 (1977) hergestellt werden. Die Verbindungen IX werden beispielsweise aus den Verbindungen II durch Methylierung, Oxydation und anschließende Deprotonierung - z.B. mit Alkalimetallhydriden oder - alkoholaten oder üblichen metallorganischen Verbindungen - erhalten. Die Verbindungen X werden in Anlehnung an Z. Talik, Roczniki Chem. 35, 475 (1961) hergestellt.

Die folgenden Beispiele erläutern die Erfindung näher ohne sie einzuschränken. Schmp. bedeutet Schmelzpunkt, "Ether" steht für Diethylether, "Essigester" bedeutet Essigsäureethylester. Für Stunde(n) wird die Abkürzung h verwendet, für Minuten Min..

Beispiele zur Ausführung der Erfindung

1. 4-Trifluormethyl-2-[(4-methoxy-2-pyridylmethyl)sulfinyl-(1H)-benzimidazol

Zu einer auf -30°C gekühlten Lösung von 3,0 g (0,0088 Mol) 4-Trifluormethyl-2-[(4-methoxy-2-pyridylmethyl)thio]-(1H)-benzimidazol in 50 ml Methylenchlorid werden 2,3 g käufliche 85%ige m-Chlorperoxybenzoesäure, gelöst in 30 ml Methylenchlorid, innerhalb von 45 Min. zugetropft. Danach rührt man noch weitere 60 Min. und läßt anschließend die Temperatur auf 0°C steigen. Nach Waschen mit gesättigter wäßriger Natriumcarbonat-Lösung (30 ml) wird über Natriumsulfat getrocknet und das Lösungsmittel im Vakuum abgezogen. Der verbleibende feste Rückstand wird nacheinander aus Isopropanol und Acetonitril umkristallisiert. Ausbeute 1,6 g vom Schmp. 150-151°C.

2. 5-Trifluormethyl-2-[(4-methoxy-2-pyridylmethyl)sulfinyl]-(1H)-benzimidazol

Analog Beispiel 1 werden 0,1 g der Titelverbindung vom Schmp. 166°C (unter Zersetzung, zweimal aus Isopropanol umkristallisiert), ausgehend von 0,3 g (0,92 mMol) 5-Trifluormethyl-2-[(4-methoxy-2-pyridylmethyl)-thio]-(1H)-benzimidazol und 0,3 g 85 %iger m-Chlorperbenzoesäure, erhalten.

3. 5-Trifluormethyl-2-[(4-methoxy-3-methyl-2-pyridylmethyl)sulfinyl]-(1H)-benzimidazol

Analog Beispiel 1 werden aus 3,7 g (0,01 Mol) 5-Trifluormethyl-2-[(4-methoxy-3-methyl-2-pyridylmethyl)thio]-(1H)-benzimidazol und 3,3 g 85%iger m-Chlorperoxybenzoesäure 2,9 g der Titelverbindung vom Schmp. 225-227°C (Zersetzung, aus Toluol/Ether) erhalten.

4. 5-Trifluormethyl-2-[(4-methoxy-3,5-dimethyl-2-pyridylmethyl)sulfinyl]-(1H)-benzimidazol

Analog Beispiel 1 werden 3,1 g der Titelverbindung, vom Schmp. 163°C (aus Acetonitril) ausgehend von 3,8 g (0,01 Mol) 5-Trifluormethyl-2-[(4-methoxy-3,5-dimethyl-2-pyridylmethyl)-thio]-(1H)-benzimidazol und 3,5 g 85%iger m-Chlorperoxybenzoesäure, erhalten.

5. 4-Trifluormethyl-2-[(4-methoxy-2-pyridylmethyl)thio]-(1H)-benzimidazol

5,0 g (0,023 Mol) 4-Trifluormethyl-2-mercapto-benzimidazol, gelöst in 100 ml Ethanol und 23 ml 2n Natronlauge, werden mit 4,5 g (0,023 Mol) 2-Chlormethyl-4-methoxypyridin-Hydrochlorid versetzt und 6 Stunden bei Raumtemperatur gerührt. Nach Abziehen des größten Teils des Lösungsmittels wird dreimal mit je 50 ml Ethylacetat extrahiert, mit destilliertem Wasser gewaschen, über Natriumsulfat getrocknet und das Lösungsmittel im Vakuum abgezogen. Der feste Rückstand wird aus Acetonitril umkristallisiert. 5,9 g (76%) Ausbeute vom Schmp. 146°C.

6. 5-Trifluormethyl-2-[(4-methoxy-2-pyridylmethyl)thio]-(1H)-benzimidazol

Analog Beispiel 5 erhält man aus 3,0 g (0,014 Mol) 5-Trifluormethyl-2-mercaptobenzimidazol und 2,7 g 2-Chlormethyl-4-methoxypyridin-Hydrochlorid nach dreistündigem Kochen am Rückfluß 2,7 g (59%) der Titelverbindung vom Schmp. 180-182°C (aus Acetonitril).

7. 5-Trifluormethyl-2-[(4-methoxy-3-methyl-2-pyridylmethyl)thio]-(1H)-benzimidazol

Analog zu Beispiel 5 erhält man 2,9 g der Titelverbindung vom Schmp. 148°C (aus Acetonitril) aus 2,2 g (0,01 Mol) 5-Trifluormethyl-2-mercaptobenzimidazol und 2,1 g 2-Chlormethyl-4-methoxy-3-methylpyridin-Hydrochlorid.

8. 5-Trifluormethyl-2-[(4-methoxy-3,5-dimethyl-2-pyridylmethyl)thio]-(1H)-benzimidazol

3,5 g der Titelverbindung vom Schmp. 163°C (aus Acetonitril) werden analog Beispiel 5 aus 2,2 g 5-Trifluormethyl-2-mercaptobenzimidazol und 2,2 g 2-Chlormethyl-4-methoxy-3,5-dimethylpyridin-Hydrochlorid erhalten.

9. 4-Trifluormethyl-2-[(4-methoxy-3-methyl-2-pyridylmethyl)thio]-(1H)-benzimidazol

Analog zu Beispiel 5 erhält man 3,1 g der Titelverbindung vom Schmp. 175°C (aus Isopropanol/Ether) aus 2,2 g (0,01 Mol) 4-Trifluormethyl-2-mercaptobenzimidazol und 2,1 g 2-Chlormethyl-4-methoxy-3-methylpyridin-Hydrochlorid.

10. 4-Trifluormethyl-2-mercaptobenzimidazol

Zur Lösung von 11,0 g (0,062 Mol) 3-Trifluormethyl-o-phenylendiamin in 100 ml Ethanol werden nacheinander 4,4 g festes Kaliumhydroxyd, gelöst in 14 ml Wasser und 6 g (0,08 Mol) Schwefelkohlenstoff zugefügt. Das Gemisch wird unter Rühren 4 Stunden am Rückfluß erhitzt (60°C Badtemperatur ). Der ausgefallene Niederschlag wird nach Abkühlen auf 10°C abgesaugt und aus Isopropanol umkristallisiert: 13,0 g (96%) der Titelverbindung vom Schmp. 315-320°C.

In analoger Weise erhält man 5-Trifluormethyl-2-mercaptobenzimidazol aus 4-Trifluormethyl-o-phenylendiamin und Schwefelkohlenstoff (Schmp. 308-310°C).

11. 2-Chlormethyl-4-methoxypyridin-Hydrochlorid

Zu einer auf -10°C gekühlten Lösung von 10 g (0,072 Mol) 2-Hydroxymethyl-4-methoxypyridin in 30 ml trockenem Chloroform werden 15 ml Thionylchlorid innerhalb von 15 Minuten zugetropft. Man läßt die Lösung auf Raumtemperatur kommen und rührt noch eineinhalb Stunden. Nach Abziehen des Lösungsmittels und des überschüssigen Thionylchlorids erhält man farblose Kristalle, die aus Isopropanol umkristallisiert werden [12,1 g (87%), Schmp. 140-141°C, Zersetzung].

In analoger Weise erhält man durch Umsetzung von 2-Hydroxymethyl-4-methoxy-3-methylpyridin bzw. 2-Hydroxymethyl-4-methoxy-3,5-dimethylpyridin mit Thionylchlorid 2-Chlormethyl-4-methoxy-3-methylpyridin-Hydrochlorid (Schmp. 151-153°C, aus Isopropanol/Ether) bzw. 2-Chlormethyl-4-methoxy-3,5-dimethylpyridin-Hydrochlorid (Schmp. 128°C, aus Isopropanol/Ether).

Die Verbindungen 2-Hydroxymethyl-4-methoxypyridin und 2-Hydroxymethyl-4-methoxy-3-methylpyridin werden in Anlehnung an bzw. nach Vorschrift von O.E. Schulz und S.Fedders, Arch. Pharm. (Weinheim) 310, 128 (1977) erhalten. Das benötigte Vorprodukt 2,3-Dimethyl-4-nitropyridin-N-oxid wird gemäß H.C. Brown, S. Johnson und H. Podall, J. Am. Chem. Soc. 76, 5556 (1954) hergestellt.

12. 2-Hydroxymethyl-4-methoxy-3,5-dimethylpyridin-Hydrochlorid

18 g 2,3,5-Trimethylpyridin [F. Bohlmann, A. Englisch, J. Politt, H. Sander und W. Weise, Chem. Ber. 88, 1831 (1955)] und 17 ml 30%iges Wasserstoffperoxid werden in 80 ml Eisessig 2,5 h bei 100°C erwärmt. Danach setzt man weitere 10 ml 30%iges Wasserstoffperoxid zu und hält die Temperatur weitere 8 h. Das Gemisch wird anschließend im Wasserstrahlvakuum auf das halbe Volumen eingeengt und einem Peroxidtest unterzogen. Bei Peroxidfreiheit wird alles Lösungsmittel im Vakuum abgezogen und der Rückstand im Hochvakuum destilliert. Bei 95-98°C und 0,01 Torr (1,33 Pa) gehen 19,2 g (95%) 2,3,5-Trimethylpyridin-N-oxid über.

5,0 g hiervon werden bei Raumtemperatur in einem Gemisch aus 7 ml rauchender Salpetersäure und 7 ml konzentrierter Schwefelsäure gelöst und 18 Stunden bei 40°C Badtemperatur erwärmt. Danach gießt man auf Eiswasser und stellt mit starker Natronlauge unter Kühlung alkalisch. Die Extraktion des Gemisches mit Essigsäureethylester ergibt nach Entfernung des Lösungsmittels im Vakuum rohes 2.3.5-Trimethyl-4-nitropyridin-N-oxid, das ohne weitere Reinigung in 20 ml trockenem Methanol gelöst wird. Zu dieser Lösung gibt man 4,7 ml käufliches 30%iges Natriummethoxid in Methanol und erwärmt 12 h auf 50°C. Danach wird das Lösungsmittel abgezogen; der Rückstand wird in wenig Wasser aufgenommen und mit Essigsäureethylester extrahiert. Nach Abziehen des Lösungsmittels wird das als Öl zurückbleibende rohe 4-Methoxy-2.3.5-trimethylpyridin-N-oxid ohne weitere Reinigung in 20 ml 100°C heißes Essigsäureanhydrid gegossen und 1 Stunde bei dieser Temperatur erwärmt. Danach wird im Vakuum eingeengt und ohne weitere Reinigung in 20 ml 10%iger wässriger Salzsäure aufgenommen und 2,5 h bei 50°C gerührt. Im Vakuum wird auf das halbe

Volumen eingeengt, mit Kaliumcarbonat alkalisch gestellt und mit Essigsäureethylester extrahiert. Die vereinigten Extrakte werden über Natriumsulfat getrocknet; das Lösungsmittel wird im Vakuum abgezogen. Der ölige Rückstand wird in 50 ml Ethylmethylketon gelöst und mit etherischer Salzsäure bis zur quantitativen Ausfällung versetzt. Der Niederschlag wird aus Dioxan mit wenig Isopropanol umkristallisiert. Man erhält 3,1 g der Titelverbindung vom Schmp. 126°C.

13. <u>5-Trifluormethyl-2-[(4-methoxy-2-pyridylmethyl)thio]-(1H)-benzimidazol</u>

Eine Lösung von 2,25 g (10,3 mmol) 5-Trifluormethyl-2-mercapto-benzimidazol in 19 ml Isopropanol und eine heiße Lösung von 2,06 g (10,6 mmol) 2-Chlormethyl-4-methoxypyridin-Hydrochlorid in 20 ml Isopropanol werden vereinigt und 4 Stunden bei 100°C gerührt. Man kühlt im Eisbad und erhält 4,21 g (99%) 5-Trifluormethyl-2-[(4-methoxy-2-pyridylmethyl)thio]-(1H)-benzimidazol-Dihydrochlorid vom Schmp. 195-197°C (Zersetzung). Eine Lösung von 4,66 g (11,3 mmol) des Dihydrochlorids in 13 ml Wasser wird mit Aktivkohle geklärt und mit 17 ml 2n Kaliumbicarbonatlösung, die etwas Isopropanol enthält, bei Raumtemperatur langsam die freie Base gefällt. Man erhält 3.7 g (96%) der Titelverbindung vom Schmp. 182-184°C.

14. <u>5-Trifluormethyl-2-[(4-methoxy-2-pyridylmethyl)sulfinyl]-(1H)-benzimidazol</u>

Zu einer 10°C warmen Lösung von 16,2 g (0,048 Mol) 5-Trifluormethyl-2-[(4-methoxy-2-pyridylmethyl)thio]-(1H)-benzimidazol in 960 ml Ethylacetat werden unter kräftigem Rühren 600 ml einer ca. 1,5%igen wäßrigen Natriumhypochlorit-Lösung innerhalb von 3 Minuten zugetropft, wobei die Temperatur zwischen 10°und 15°C gehalten wird. Anschließend wird noch 2 Minuten gerührt und die organische Phase abgetrennt und mit 100 ml destilliertem Wasser gewaschen. Die wäßrige Phase wird mit Eisessig auf pH 7-8 eingestellt und dreimal mit Ethylacetat extrahiert. Die Extrakte werden mit Wasser gewaschen und mit der obigen Ethylacetat-Lösung vereinigt. Danach wird im Vakuum bis auf ein Volumen von 100 ml eingeengt und im Eisbad gekühlt. Die hierbei ausgefallene

Titelverbindung wird abgesaugt und mit kaltem Ethylacetat gewaschen. Ausbeute 13,2 g vom Schmp. 166°C (unter Zersetzung).

15. 5-Trifluormethyl-2-[(4-methoxy-2-pyridylmethyl)sulfinyl]-(1H)-benzimidazol

Eine Suspension von 20,0 g (0,092 Mol) 5-Trifluormethyl-2-mercapto-benzimidazol und 13,1 g (0,092 Mol) Methyliodid in 200 ml Ethanol wird 3 Stunden am Rückfluß gekocht. Nach Abziehen des Lösungsmittels im Vakuum wird aus Acetonitril umkristallisiert. Man erhält 17,5 g (82%) 5-Trifluormethyl-2-methylthiobenzimidazol vom Schmp. 145-146°C. 3 g (0,013 Mol) hiervon werden in 50 ml Methylenchlorid suspendiert. Bei -20°C wird eine Lösung von 2,6 g (0,013 Mol) käuflicher 85%iger m-Chlorperoxybenzoesäure in 20 ml Methylenchlorid innerhalb von 30 Minuten zugetropft. Nach einer weiteren Stunde Rühren wird zweimal mit je 20 ml einer wäßrigen 10%igen Kaliumcarbonat-Lösung extrahiert. Das Lösungsmittel wird im Vakuum abgezogen und der verbleibende kristalline Rückstand aus Acetonitril umkristallisiert. Man erhält 1,8 g 5-Trifluormethyl-2-methylsulfinyl-benzimidazol vom Schmp. 135°C. Die Deprotonierung dieser Verbindung mit n-Butyllithium und die Umsetzung mit 2-Chlor-4-methoxypyridin in trockenem Benzol liefert die Titelverbindung vom Schmp. 166°C (Zersetzung).

16. 5-Trifluormethyl-2-[(4-methoxy-3,5-dimethyl-2-pyridylmethyl)thio]-(1H)-benzimidazol

2,27 g 2-[2-(4-Methoxy-3,5-dimethyl)pyridylmethylthio]ameisensäure und 1,76 g 4-Trifluormethyl-o-phenylendiamin werden 2 Stunden in 20 ml 4n HCl am Rückfluß gekocht. Nach dem Abkühlen wird mit wäßriger Ammoniak-Lösung neutralisiert und mit Ethylacetat extrahiert. Nach Abziehen des Lösungsmittels erhält man einen festen Rückstand, der aus Acetonitril umkristallisiert wird. 3,1 g der Titelverbindung vom Schmp. 163°C.

17. 5-Trifluormethyl-2-[(4-methoxy-2-pyridylmethyl)thio]-(1H)-benzimidazol

22,1 g (0,1 Mol) 5-Trifluormethyl-2-chlor-benzimidazol werden zusammen mit 15,7 g (0,11 Mol) 4-Methoxy-2-thiomethylpyridin in 250 ml Isopropanol 10 Stunden unter Rückfluß erhitzt. Danach wird das Lösungsmittel im Vakuum größtenteils abgezogen und der Rückstand mit 500 ml Eiswasser versetzt. Die Titelverbindung wird abgesaugt und aus Acetonitril umkristallisiert. Schmp. 180-182°C.

18. 5-Trifluormethyl-2-[(4-methoxy-2-pyridylmethyl)sulfinyl]-(1H)-benzimidazol Calciumsalz

Zur Lösung von 1,1 g 5-Trifluormethyl-2-[(4-methoxy-2-pyridylmethyl)-sulfinyl]-(1H)-benzimidazol in Dioxan werden unter Inertgas 0,09g (0,003 Mol) 80%iges Natriumhydrid (in Paraffin) zugefügt. Nach dem Abklingen der Gasentwicklung wird das Lösungsmittel im Vakuum abgezogen. Der Rückstand (5-Trifluormethyl-2-[(4-methoxy-2-pyridylmethyl)-sulfinyl]-(1H)benzimidazol Natriumsalz) wird in wenig Wasser gelöst und mit 1%iger Calciumchlorid-Lösung in Wasser versetzt. Der entstehende Niederschlag wird abgesaugt und im Hochvakuum mehrere Stunden getrocknet. Schmp. 176°C unter Zersetzung.

Gewerbliche Anwendbarkeit

Die substituierten Benzimidazole der allgemeinen Formel I und ihre Salze besitzen wertvolle pharmakologische Eigenschaften, die sie gewerblich verwertbar machen. Sie hemmen deutlich die Magensäuresekretion von Warmblütern und weisen darüberhinaus eine ausgezeichnete Magen- und Darmschutzwirkung bei Warmblütern auf. Diese Magen- und Darmschutzwirkung wird bereits bei der Verabreichung von Dosen beobachtet, die unterhalb der säuresekretionshemmenden Dosen liegen.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z.B. Ethanol), Magensäure oder Streßsituationen verursacht werden können.

Ein weiterer Vorteil der erfindungsgemäßen Verbindungen ist ihre vergleichsweise große chemische Stabilität.

In ihren ausgezeichneten Eigenschaften erweisen sich die erfindungsgemäßen Verbindungen überraschenderweise den aus dem Stand der Technik bekannten Verbindungen deutlich überlegen. Aufgrund dieser Eigenschaften sind die substituierten Benzimidazole und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Ein weiterer Gegenstand der Erfindung sind daher die erfindungsgemäßen Verbindungen zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere substituierte Benzimidazole der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragées, Kapseln, Suppositorien, Emulsionen, Suspensionen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale und die intravenöse Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 7, insbesondere 0,1 bis 2 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen

optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Die Formulierung der Wirkstoffe kann z.B. auf folgende Weise erfolgen:

a) Tabletten mit 40 mg Wirkstoff

20 kg 4-Trifluormethyl-2-[(4-methoxy-2-pyridylmethyl)thio]-(1H)-benzimidazol, 40 kg Milchzucker, 26 kg Maisstärke und 3 kg Polyvinylpyrrolidon werden mit ca. 20 Liter Wasser befeuchtet und durch ein Sieb mit 1,25 mm Maschenweite granuliert. Das Granulat wird im Wirbelschichttrockner bis zu einer relativen Feuchte von 50-60 % getrocknet und dann mit 8 kg Natriumcarboxymethylcellulose, 2 kg Talkum und 1 kg Magnesiumstearat versetzt. Das fertige Granulat wird zu Tabletten à 200 mg und 8 mm Durchmesser gepreßt.

b) Kapseln mit einem Wirkstoffgehalt von 30 mg

300 g 5-Trifluormethyl-2-[(4-methoxy-3-methyl-2-pyridylmethyl)thio]-(1H)-benzimidazol, 695 g mikrokristalline Cellulose und 5 g amorphe Kieselsäure werden feingepulvert, gut vermischt und in Hartgelatinekapseln Größe 4 abgefüllt.

c) Kapseln mit einem Wirkstoffgehalt von 10 mg

100 g 5-Trifluormethyl-2-[(4-methoxy-2-pyridylmethyl)sulfinyl]-(1H)-benzimidazol, 895 g mikrokristalline Cellulose und 5 g amorphe Kieselsäure werden feingepulvert, gut vermischt und in Hartgelatinekapseln Größe 4 abgefüllt.

d) Ampullen enthaltend 30 mg 5-Trifluormethyl-2-[(4-methoxy-3-methyl-2-pyridylmethyl)thio]-(1H)-benzimidazol
60 g 5-Trifluormethyl-2-[(4-methoxy-3-methyl-2-pyridylmethyl)thio]-(1H)-benzimidazol werden in 9 kg destilliertem Wasser suspendiert und durch langsame Zugabe von 1n Salzsäure gelöst, bis die klare Lösung einen pH von 5 erreicht hat (ca. 171 ml). Nach Zugabe von 90 g Natriumchlorid und 5 g Natriumdisulfit wird mit destilliertem Wasser auf 10 l aufgefüllt. Die Lösung wird sterilfiltriert und in 5 ml-Ampullen abgefüllt. Diese werden bei 121°C 20 Minuten sterilisiert.

e) Ampullen enthaltend 30 mg 5-Trifluormethyl-2-[(4-methoxy-2-pyridylmethyl)sulfinyl]-(1H)-benzimidazol
3 g 5-Trifluormethyl-2-[(4-methoxy-2-pyridylmethyl)sulfinyl]-(1H)-benzimidazol werden unter Rühren in einer Lösung von 6 g Natriumcarbonat in 90 ml destilliertem Wasser gelöst. Die entstandene Lösung wird mit destilliertem Wasser auf 100 ml aufgefüllt und sterilfiltriert. Von dieser Lösung wird je 1 ml in ein 10 ml Vial eindosiert und lyophilisiert.

## Pharmakologie

Die ausgezeichnete Magenschutzwirkung und die magensekretionshemmende Wirkung der erfindungsgemäßen substituierten Benzimidazole läßt sich tierexperimentell am Modell Shay-Ratte nachweisen. Die erfindungsgemäßen Verbindungen erweisen sich dem Stand der Technik hinsichtlich der Magenschutzwirkung und der magensekretionshemmenden Wirkung um ein Mehrfaches überlegen. Die untersuchten Verbindungen sind wie folgt mit Nummern versehen worden:

| lfd. Nr. | Name der Verbindung |
|---|---|
| 1 | Omeprazol* (INN, EP-B1 0 005 129) |
| 2 | 5-Trifluormethyl-2-[(4-methoxy-2-pyridylmethyl)sulfinyl]-(1H)-benzimidazol |
| 3 | 5-Trifluormethyl-2-[(4-methoxy-3-methyl-2-pyridylmethyl)-sulfinyl]-benzimidazol |
| 4 | 5-Trifluormethyl-2-[(4-methoxy-3,5-dimethyl-2-pyridylmethyl)sulfinyl]-(1H)-benzimidazol |
| 5 | 4-Methyl-2-[(2-pyridylmethyl)thio]-(1H)-benzimidazol (DE-OS 25 48 340, USP 4,045,564) |
| 6 | 5-Trifluormethyl-2-[(4-methoxy-2-pyridylmethyl)thio]-(1H)-benzimidazol |
| 7 | 5-Trifluormethyl-2-[(4-methoxy-3-methyl-2-pyridylmethyl)-thio]-(1H)-benzimidazol |
| 8 | 4-Trifluormethyl-2-[(4-methoxy-3-methyl-2-pyridylmethyl)-thio]-(1H)-benzimidazol |

* Omeprazol = 5-Methoxy-2-[(4-methoxy-3,5-dimethyl-2-pyridylmethyl)-sulfinyl]-(1H)-benzimidazol

Der Einfluß der erfindungsgemäßen Verbindungen auf die durch Pylorusligatur (4h; sog. Shay-Ratte) und orale Gabe von 100 mg/kg Acetylsalicylsäure ausgelöste Magenläsionsbildung sowie die Magensekretion (HCl) währen 4 h bei der Ratte ist in der folgenden Tabelle dargestellt.

Magenschutzwirkung und Magensekretionshemmung, Vergleich erfindungsgemäßer Verbindungen mit dem Stand der Technik*

| Lfd. Nr. | Dosis [mg/kg] p.o. | Magenschutzwirkung (Ratte) Hemmung des Läsionsindexes % | ED25+) [mg/kg, p.o.] | ED50+) [mg/kg, p.o.] | Magensekretion (Ratte) % Hemmung der HCl-Sekretion | ED25+) [mg/kg, p.o.] | ED50+) [mg/kg, p.o.] |
|---|---|---|---|---|---|---|---|
| 1 * | 0.1<br>0.3<br>1.0<br>3.0 | +1<br>12<br>27<br>58 | 0.9 | 2.3 | 5<br>9<br>15<br>22 | ≳ 3.0 | - |
| 2 | 0.1<br>0.3<br>1.0<br>3.0 | 6<br>29<br>88<br>100 | 0.24 | 0.45 | 0<br>18<br>60<br>84 | 0.35 | 0.75 |
| 3 | 0.3<br>1.0<br>10.0 | 25<br>98<br>100 | 0.3 | 0.45 | 40<br>88<br>92 | < 0.3 | 0.4 |
| 4 | 0.1<br>0.3<br>1.0<br>3.0 | 1<br>30<br>69<br>89 | 0.24 | 0.55 | +5<br>10<br>50<br>75 | 0.48 | 1.0 |
| 5 * | 10.0<br>30.0<br>100.0 | 5<br>51<br>61 | 16.0 | 30.0 | 0<br>0<br>15 | - | > 100 |
| 6 | 1.0<br>3.0 | 27<br>97 | ~1.0 | 1.4 | 15<br>70 | 1.2 | 2.0 |
| 7 | 0.3<br>1.0 | 30<br>94 | ≲0.3 | 0.5 | 26<br>85 | 0.3 | 0.5 |
| 8 | 1.0<br>3.0 | 50<br>63 | ≲1.0 | 1.0 | 12<br>50 | 1.5 | 3.0 |

+) $ED_{25}$ bzw. $ED_{50}$ = Dosis, die den Läsionsindex bzw. die HCl-Sekretion ($\Sigma$4 h) des Rattenmagens bei der behandelten Gruppe gegenüber der Kontrollgruppe um 25 bzw. 50 % mindert.

Die Prüfung der antiulcerogenen Wirkung erfolgte nach der Methode der sogenannten Shay-Ratte:
Die Ulcusprovokation erfolgt bei 24 Stunden nüchtern gehaltenen Ratten (weiblich, 180 - 200 g, 4 Tiere je Käfig auf hohem Gitterrost) durch Pylorusligatur (unter Diethylethernarkose) und orale Applikation von 100 mg/10 ml/kg Acetylsalicylsäure. Die zu prüfenden Substanzen werden oral (10 ml/kg) eine Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen)und später sein HCl-Gehalt (Titration mit Natronlauge) bestimmt wurde; mit einem Stereomikroskop werden bei 10-facher Vergrößerung Anzahl und Größe (=Durchmesser) vorhandener Ulcera ermittelt. Das Produkt aus Schweregrad (gemäß nachfolgender Punkteskala) und Anzahl der Ulcera dient als individueller Läsionsindex.

Punkteskala:

| | | |
|---|---|---|
| keine Ulcera | | 0 |
| Ulcusdurchmesser | 0,1 - 1,4 mm | 1 |
| | 1,5 - 2,4 mm | 2 |
| | 2,5 - 3,4 mm | 3 |
| | 3,5 - 4,4 mm | 4 |
| | 4,5 - 5,4 mm | 5 |
| | $>$ 5,5 mm | 6 |

Als Maß für den antiulcerogenen Effekt dient die Minderung des mittleren Läsionsindex jeder behandelten Gruppe gegenüber dem der Kontrollgruppe (=100 %). Die $ED_{25}$ bzw. $ED_{50}$ bezeichnen diejenigen Dosen, die den mittleren Läsionsindex bzw. die HCl-Sekretion gegenüber der Kontrolle um 25% bzw. 50% mindern.

Toxizität

Die dosis tolerata aller geprüften Verbindungen liegt oberhalb von 1000 mg/kg [p.o.] bei der Maus.

## Patentansprüche

(für alle benannten Vertragsstaaten außer Österreich)

1. Substituierte Benzimidazole der allgemeinen Formel I

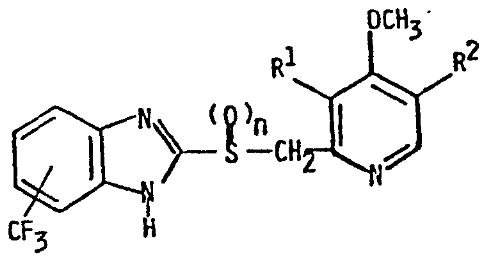

(I),

worin
$R^1$ Wasserstoff oder Methyl und
$R^2$ Wasserstoff oder Methyl bedeutet und
n für die Zahlen 0 oder 1 steht,
und die Salze dieser Verbindungen.

2. Substituierte Benzimidazole der allgemeinen Formel $I^a$

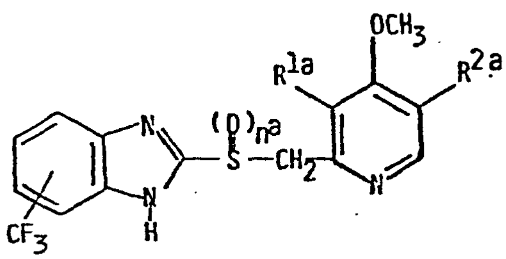

($I^a$),

worin
$R^{1a}$ Wasserstoff oder Methyl,
$R^{2a}$ Wasserstoff oder Methyl und
$n^a$ die Zahl 0 bedeutet,
und die Salze dieser Verbindungen.

3. Substituierte Benzimidazole der allgemeinen Formel $I^b$

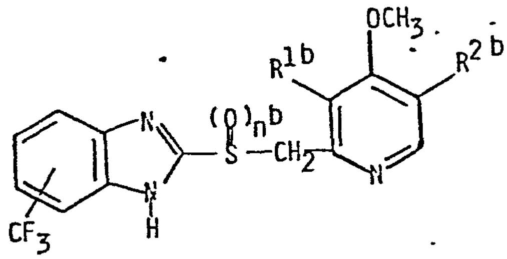

$(I^b)$,

worin
$R^{1b}$ Wasserstoff oder Methyl,
$R^{2b}$ Wasserstoff oder Methyl und
$n^b$ die Zahl 1 bedeutet,
und die Salze dieser Verbindungen.

4. Ein substituiertes Benzimidazol nach Anspruch 2, ausgewählt aus der Gruppe bestehend aus
4-Trifluormethyl-2-[(4-methoxy-2-pyridylmethyl)thio]-(1H)-benzimidazol
4-Trifluormethyl-2-[(4-methoxy-3-methyl-2-pyridylmethyl)thio]-(1H)-benzimidazol
4-Trifluormethyl-2-[(4-methoxy-5-methyl-2-pyridylmethyl)thio]-(1H)-benzimidazol
4-Trifluormethyl-2-[(4-methoxy-3,5-dimethyl-2-pyridylmethyl)thio]-(1H)-benzimidazol
5-Trifluormethyl-2-[(4-methoxy-2-pyridylmethyl)thio]-(1H)-benzimidazol
5-Trifluormethyl-2-[(4-methoxy-3-methyl-2-pyridylmethyl)thio]-(1H)-benzimidazol
5-Trifluormethyl-2-[(4-methoxy-5-methyl-2-pyridylmethyl)thio]-(1H)-benzimidazol
5-Trifluormethyl-2-[(2-methoxy-3,5-dimethyl-2-pyridylmethyl)thio]-(1H)-benzimidazol
oder ein Salz davon.

5. Ein substituiertes Benzimidazol nach Anspruch 3, ausgewählt aus der Gruppe bestehend aus
4-Trifluormethyl-2-[(4-methoxy-2-pyridylmethyl)sulfinyl]-(1H)-benzimidazol
4-Trifluormethyl-2-[(4-methoxy-3-methyl-2-pyridylmethyl)sulfinyl]-(1H)-benzimidazol

4-Trifluormethyl-2-[(4-methoxy-5-methyl-2-pyridylmethyl)sulfinyl]-(1H) benzimidazol

4-Trifluormethyl-2-[(4-methoxy-3,5-dimethyl-2-pyridylmethyl)sulfinyl]-(1H)-benzimidazol

5-Trifluormethyl-2-[(4-methoxy-2-pyridylmethyl)sulfinyl]-(1H)-benzimidazol

5-Trifluormethyl-2-[(4-methoxy-3-methyl-2-pyridylmethyl)sulfinyl]-(1H)-benzimidazol

5-Trifluormethyl-2-[(4-methoxy-5-methyl-2-pyridylmethyl)sulfinyl]-(1H)-benzimidazol

5-Trifluormethyl-2-[(4-methoxy-3,5-dimethyl-2-pyridylmethyl)sulfinyl]-(1H)-benzimidazol

oder ein Salz davon.

6. Verfahren zur Herstellung substituierter Benzimidazole der Formel I nach Anspruch 1 und ihrer Salze,
dadurch gekennzeichnet, daß man

a) Mercaptobenzimidazole der Formel II mit Picolinderivaten III,

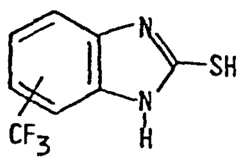 (II), 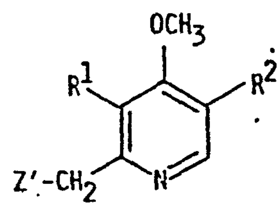 (III),

oder

b) Benzimidazole der Formel IV mit Mercaptopicolinen V,

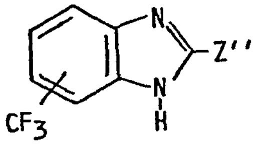 (IV), 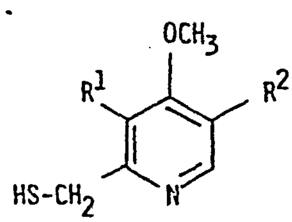 (V),

oder

c) o-Phenylendiamine der Formel VI mit Ameisensäurederivaten VII

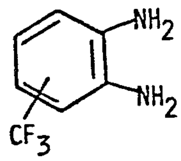 (VI), 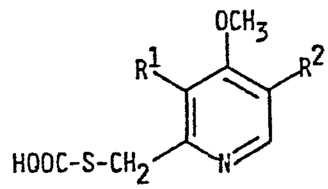 (VII),

umsetzt und gegebenenfalls anschließend die nach a), b) oder c) erhaltenen 2-Benzimidazolyl-2-pyridyl-sulfide der Formel VIII

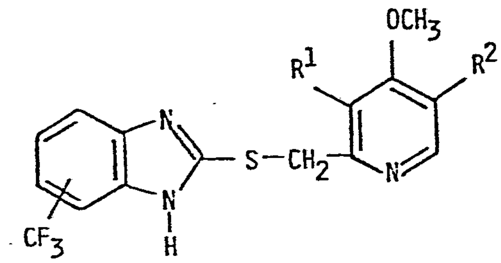 (VIII),

oxidiert und/oder in die Salze überführt,
oder daß man
d) Benzimidazole der Formel IX mit Pyridinderivaten X

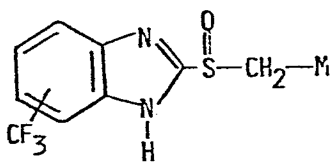 (IX), 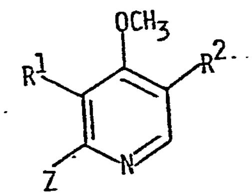 (X),

oder
e) Sulfinylderivate der Formel XI mit 2-Picolinderivaten XII

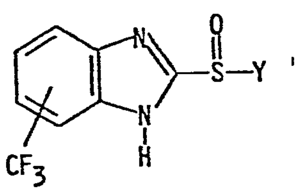 (XI) 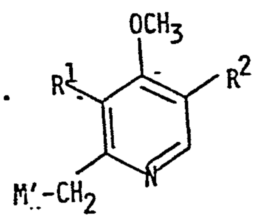 (XII)

umsetzt und gegebenenfalls anschließend in die Salze überführt, wobei Y, Z, Z' und Z'' geeignete Abgangsgruppen darstellen, M für ein Alkalimetallatom (Li, Na oder K) steht, M' für das Äquivalent eines Metallatoms steht und $R^1$, $R^2$ und n die in Anspruch 1 angegebenen Bedeutungen haben.

7. Substituierte Benzimidazole nach Anspruch 1 und ihre pharmakologisch verträglichen Salze zur Anwendung bei der Behandlung und Prophylaxe von Krankheiten des Magens und Darms und solcher Krankheiten, die auf einer erhöhten Magensäuresekretion beruhen.

8. Arzneimittel enthaltend ein oder mehrere substituierte Benzimidazole nach einem oder mehreren der Ansprüche 1 bis 5 und/oder ihre pharmakologisch verträglichen Salze.

## Patentansprüche für Österreich

1. Verfahren zur Herstellung substituierter Benzimidazole der allgemeinen Formel I

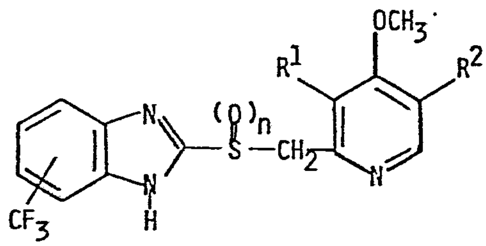

(I),

worin
$R^1$ Wasserstoff oder Methyl und
$R^2$ Wasserstoff oder Methyl bedeutet und
n für die Zahlen 0 oder 1 steht,
und ihrer Salze,
dadurch gekennzeichnet, daß man
a) Mercaptobenzimidazole der Formel II mit Picolinderivaten III,

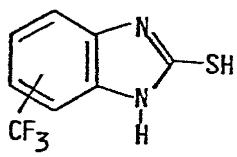

(II),

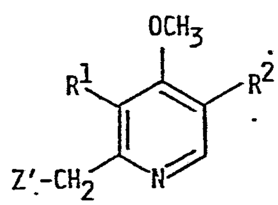

(III),

oder
b) Benzimidazole der Formel IV mit Mercaptopicolinen V,

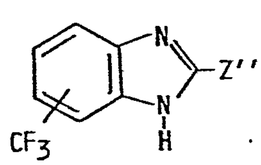

(IV),

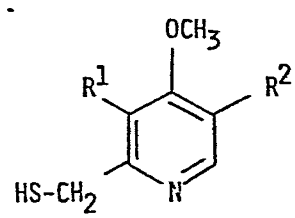

(V),

oder

c) o-Phenylendiamine der Formel VI mit Ameisensäurederivaten VII

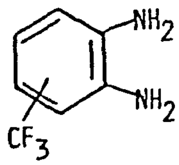 (VI), 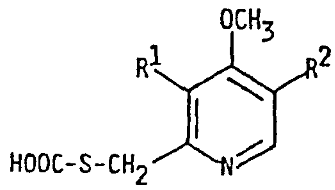 (VII),

umsetzt und gegebenenfalls anschließend die nach a), b) oder c) erhaltenen 2-Benzimidazolyl-2-pyridyl-sulfide der Formel VIII

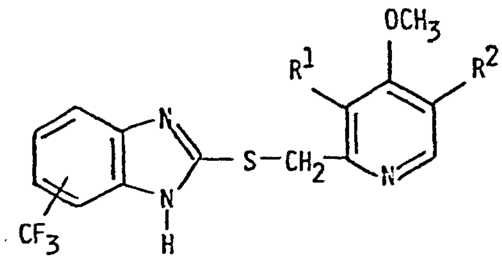 (VIII),

oxidiert und/oder in die Salze überführt,
oder daß man
d) Benzimidazole der Formel IX mit Pyridinderivaten X

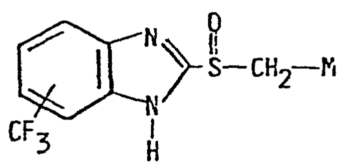 (IX), 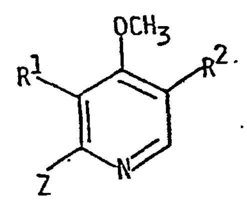 (X),

oder
e) Sulfinylderivate der Formel XI mit 2-Picolinderivaten XII

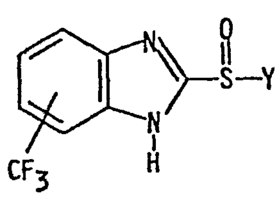 (XI) 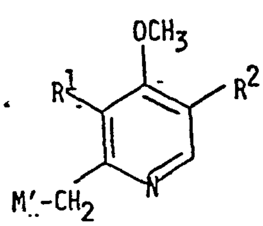 (XII)

umsetzt und gegebenenfalls anschließend in die Salze überführt, wobei Y, Z, Z' und Z'' geeignete Abgangsgruppen darstellen, M für ein Alkalimetallatom (Li, Na oder K) steht, M' für das Äquivalent eines Metallatoms steht und $R^1$, $R^2$ und n die oben angegebenen Bedeutungen haben.

2. Verfahren nach Anspruch 1 zur Herstellung substituierter Benzimidazole der allgemeinen Formel I, worin $R^1$ Wasserstoff oder Methyl und $R^2$ Wasserstoff oder Methyl bedeutet und n für die Zahl 0 steht, und ihrer Salze, dadurch gekennzeichnet, daß man Mercaptobenzimidazole der Formel II mit Picolinderivaten III, oder Benzimidazole der Formel IV mit Mercaptopicolinen V, oder o-Phenylendiamine der Formel VI mit Ameisensäurederivaten VII umsetzt und gegebenenfalls anschließend die erhaltenen Produkte in die Salze überführt.

3. Verfahren nach Anspruch 1 zur Herstellung substituierter Benzimidazole der allgemeinen Formel I, worin $R^1$ Wasserstoff oder Methyl und $R^2$ Wasserstoff oder Methyl bedeutet und n für die Zahl 1 steht, und ihrer Salze, dadurch gekennzeichnet, daß man 2-Benzimidazolyl-2-pyridyl-sulfide der Formel VIII oxidiert, oder Benzimidazole der Formel IX mit Pyridinderivaten X, oder Sulfinylderivate der Formel XI mit 2-Picolinderivaten XII umsetzt und gegebenenfalls anschließend die erhaltenen Produkte in die Salze überführt.

4. Verfahren nach Anspruch 2, dadurch gekennzeichnet, daß man
4-Trifluormethyl-2-[(4-methoxy-2-pyridylmethyl)thio]-(1H)-benzimidazol,
4-Trifluormethyl-2-[(4-methoxy-3-methyl-2-pyridylmethyl)thio]-(1H)-benzimidazol,
4-Trifluormethyl-2-[(4-methoxy-5-methyl-2-pyridylmethyl)thio]-(1H)-benzimidazol,
4-Trifluormethyl-2-[(4-methoxy-3,5-dimethyl-2-pyridylmethyl)thio]-(1H)-benzimidazol,
5-Trifluormethyl-2-[(4-methoxy-2-pyridylmethyl)thio]-(1H)-benzimidazol,
5-Trifluormethyl-2-[(4-methoxy-3-methyl-2-pyridylmethyl)thio]-(1H)-benzimidazol,
5-Trifluormethyl-2-[(4-methoxy-5-methyl-2-pyridylmethyl)thio]-(1H)-benzimidazol oder
5-Trifluormethyl-2-[(2-methoxy-3,5-dimethyl-2-pyridylmethyl)thio]-(1H)-benzimidazol
oder ein Salz davon herstellt.

5. Verfahren nach Anspruch 3, dadurch gekennzeichnet, daß man 4-Trifluormethyl-2-[(4-methoxy-2-pyridylmethyl)sulfinyl]-(1H)-benzimidazol, 4-Trifluormethyl-2-[(4-methoxy-3-methyl-2-pyridylmethyl)sulfinyl]-(1H)-benzimidazol, 4-Trifluormethyl-2-[(4-methoxy-5-methyl-2-pyridylmethyl)sulfinyl]-(1H) benzimidazol, 4-Trifluormethyl-2-[(4-methoxy-3,5-dimethyl-2-pyridylmethyl)sulfinyl]-(1H)-benzimidazol, 5-Trifluormethyl-2-[(4-methoxy-2-pyridylmethyl)sulfinyl]-(1H)-benzimidazol, 5-Trifluormethyl-2-[(4-methoxy-3-methyl-2-pyridylmethyl)sulfinyl]-(1H)-benzimidazol, 5-Trifluormethyl-2-[(4-methoxy-5-methyl-2-pyridylmethyl)sulfinyl]-(1H)-benzimidazol oder 5-Trifluormethyl-2-[(4-methoxy-3,5-dimethyl-2-pyridylmethyl)sulfinyl]-(1H)-benzimidazol oder ein Salz davon herstellt.

6. Verfahren zur Herstellung substituierter Benzimidazole der allgemeinen Formel $I^a$

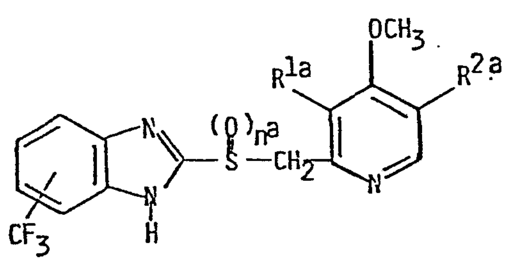

$(I^a)$,

worin
$R^{1a}$ Wasserstoff oder Methyl,
$R^{2a}$ Wasserstoff oder Methyl und
$n^a$ die Zahl 0 bedeutet,
und ihrer Salze,
dadurch gekennzeichnet, daß man

Mercaptobenzimidazole der Formel II mit Picolinderivaten III,

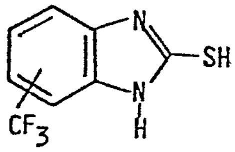

(II),

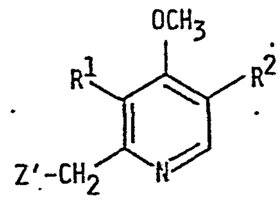

(III),

umsetzt und gegebenenfalls anschließend in die Salze überführt, wobei $R^1$ die Bedeutung von $R^{1a}$ und $R^2$ die Bedeutung von $R^{2a}$ hat und Z' eine Abgangsgruppe darstellt.

7. Verfahren zur Herstellung substituierter Benzimidazole der allgemeinen Formel $I^b$

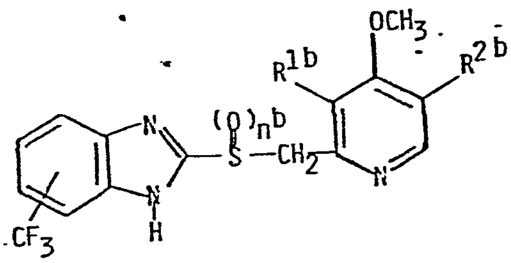

($I^b$),

worin
$R^{1b}$ Wasserstoff oder Methyl,
$R^{2b}$ Wasserstoff oder Methyl und
$n^b$ die Zahl 1 bedeutet,
und ihrer Salze,
dadurch gekennzeichnet, daß man
2-Benzimidazolyl-2-pyridyl-sulfide der Formel VIII

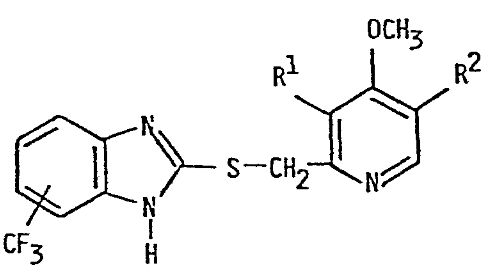

(VIII),

oxidiert und gegebenenfalls anschließend in die Salze überführt,

wobei $R^1$ die Bedeutung von $R^{1b}$ und $R^2$ die Bedeutung von $R^{2b}$ hat.

8. Verfahren nach Anspruch 6, dadurch gekennzeichnet, daß die Abgangsgruppe Z' ein Halogenatom oder eine durch Veresterung aktivierte Hydroxylgruppe darstellt.

9. Verfahren nach Anspruch 7, dadurch gekennzeichnet, daß die Oxidation mit einem Halogen oder einem Hypohalogenit durchgeführt wird.

10. Verfahren zur Herstellung neuer Arzneimittel, die zur Behandlung und Prophylaxe von Krankheiten des Magens und Darms und solcher Krankheiten, die auf einer überhöhten Magensäuresekretion beruhen, geeignet sind, dadurch gekennzeichnet, daß bei der Herstellung der Arzneimittel die gemäß Anspruch 1 erhaltenen Verbindungen der allgemeinen Formel I und/oder ihre pharmakologisch verträglichen Salze als aktive Bestandteile eingesetzt werden.

Europäisches Patentamt

# EUROPÄISCHER RECHERCHENBERICHT

0080602
Nummer der Anmeldung

EP 82 11 0054

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | FR-A-2 392 021 (AB HÄSSLE)<br>--- | | C 07 D 401/12<br>A 61 K 31/44<br>A 61 K 31/415 |
| D,A | EP-A-0 005 129 (AB HÄSSLE)<br>--- | | |
| P,D<br>A | EP-A-0 045 200 (UPJOHN)<br>----- | | |
| | | | RECHERCHIERTE SACHGEBIETE (Int. Cl. ³) |
| | | | C 07 D 401/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 09-02-1983 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument